# FASCICULE DE BREVET EUROPEEN

(11) **EP 3 291 155 B1**
(45) Date de publication et mention de la délivrance du brevet: **14.07.2021**
(21) Numéro de dépôt: 17189412.4
(22) Date de dépôt: 05.09.2017
(51) Int. Cl.: G06Q 10/08, G06Q 50/28, G01K 1/14, G01L 19/08, G01K 13/00, G08B 23/00

(54) **PROCÉDÉ DE SURVEILLANCE D'UN FLACON DE PRODUIT ALIMENTAIRE, EN PARTICULIER DE BOISSON ALCOOLISÉE**
VERFAHREN ZUR ÜBERWACHUNG EINER FLASCHCHE MIT EINEM LEBENSMITTELPRODUKT, INSBESONDERE EINEM ALKOHOLHALTIGEN GETRÄNK
METHOD FOR MONITORING A BOTTLE OF FOOD PRODUCT, IN PARTICULAR OF AN ALCOHOLIC BEVERAGE

(30) Priorité: 05.09.2016 FR 1658222
(43) Date de publication de la demande: 07.03.2018
(73) Titulaire: L'Effervescence des Marques, 59100 Roubaix (FR)
(72) Inventeur: REVILLON, Delphine, 59134 HERLIES (FR); LOONIS, Nicolas, 59134 HERLIES (FR)
(74) Mandataire: Regimbeau

(56) Documents cités:
- WO-A1-2014/195721
- US-A1- 2006 026 971

## Description

### DOMAINE TECHNIQUE GENERAL

La présente invention concerne le domaine des objets communicants.

Plus précisément, elle concerne un procédé de surveillance d'un flacon de produit alimentaire, en particulier de boisson alcoolisée.

### ETAT DE L'ART

Les boissons alcoolisées, quelle que soit leur nature, sont issues d'un processus de fermentation naturelle de produits alimentaires, à l'issue duquel elles peuvent être conservées un certain temps.

Dans le cas des vins, des champagnes et de certains spiritueux, une période d'attente pouvant durant plusieurs dizaines d'années, appelée garde, est souhaitable afin de conférer à la boisson toutes ses qualités organoleptiques et physico-chimiques.

Plus précisément, pendant cette période, la boisson continue à échanger des gaz avec l'atmosphère à travers le bouchon de la bouteille, des réactions chimiques continuent d'avoir lieu, et les arômes évoluent.

Toutefois, des conditions particulières en termes de température, humidité, luminosité, orientation de la bouteille, etc., sont nécessaires pour que ce processus de vieillissement ait lieu de façon optimale, c'est pourquoi les bouteilles sont souvent conservées dans des caves, qui présentent généralement des conditions plus favorables à la conservation que l'intérieur des habitations (température plus basse, humidité plus élevée, absence de lumière, etc.).

Mais l'utilisation d'une cave n'est pas toujours suffisante : par exemple, dans une période de forte sécheresse, si le taux d'humidité descend en dessous de 70 %, les bouchons sèchent rapidement et le vin commence à s'évaporer, causant une oxydation prématurée, ce phénomène étant aggravé si la température est trop élevée.

Pour pallier aux problèmes de conservation, sont proposées des équipements appelés caves à vin réfrigérées, qui sont des armoires dans lesquelles les conditions de stockage sont contrôlées électroniquement. Un tel équipement s'avère toutefois cher, encombrant et d'une capacité limitée, et il est dommage de ne pas se servir de sa cave si l'on en possède une.

Ainsi, sont également proposés des systèmes d'alarme de cave, qui mesurent en permanence la température et l'hygrométrie de la cave, et avertissent l'utilisateur en cas d'anomalie (c'est-à-dire de variation en dehors de conditions dites nominales) de sorte qu'il puisse déplacer ses bouteilles si les conditions de stockage se dégradent.

On connait par ailleurs des documents WO 2014/195721 et US 2006/026971 des thermomètres pour bouteille permettant de servir un vin à la bonne température.

Dans un cas comme dans l'autre, les systèmes connus apportent satisfaction, mais reste perfectibles. En effet :
- Les conditions optimales de stockage diffèrent d'une boisson à l'autre, et dépendent également de l'âge de la boisson ;
- Les effets d'une détérioration des conditions de stockage diffèrent eux aussi selon la boisson et son âge.

Il serait ainsi souhaitable de disposer d'un système évolué qui permette de surveiller indépendamment les conditions de stockage d'une pluralité de bouteilles de boissons alcoolisées, de sorte à optimiser au mieux leur vieillissement et offrir les meilleures conditions possibles de dégustation.

### PRESENTATION DE L'INVENTION

La présente invention se rapporte ainsi à un procédé de surveillance d'un flacon de produit alimentaire, et un système, selon les revendications.

### PRESENTATION DES FIGURES

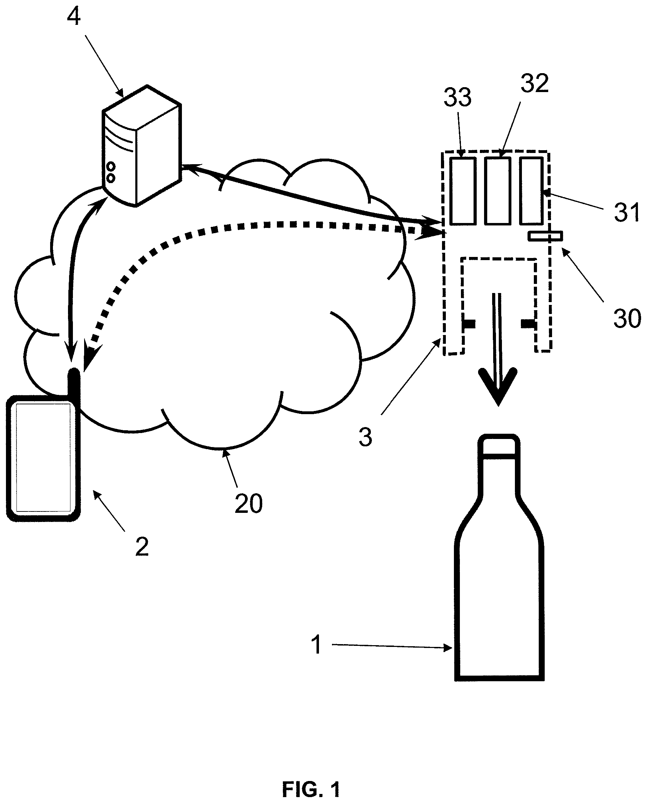

D'autres caractéristiques et avantages de la présente invention apparaîtront à la lecture de la description qui va suivre d'un mode de réalisation préférentiel. Cette description sera donnée en référence à la figure 1 annexée qui est un schéma d'une architecture pour la mise en œuvre du procédé de surveillance d'un flacon de produit alimentaire selon l'invention.

### DESCRIPTION DETAILLEE

### Architecture

En référence à la **figure 1**, l'invention propose un procédé de surveillance d'un flacon 1 de produit alimentaire, en particulier de boisson alcoolisée. Par boisson alcoolisée en entend n'importe quelle boisson issue de la fermentation alcoolique telle que vin, champagne, bière, cidre, spiritueux, etc., qui soit apte à subir un processus de vieillissement (typiquement en cave), qui peut durer plusieurs années. Le flacon 1 désigne dans ce cas typiquement une bouteille en verre bouchée par un bouchon enfoncé dans un goulot, en diverses tailles, mais on comprendra qu'il peut s'agir de tout contenant tel qu'un tonneau, une cuve ou un BIB (« Bag In box »).

On comprendra toutefois que l'invention peut également s'appliquer à d'autres produits alimentaires processus de vieillissement tels que des boissons non-alcoolisées, de la nourriture solide ou semi-liquide, le flacon 1 pouvant alors être un bocal, une verrine, une conserve, etc. Dans la suite de la présente description, on prendra l'exemple préféré d'un flacon 1 de boisson alcoolisée.

Par surveillance, on entend la vérification des conditions de stockage dudit flacon 1, et plus précisément le déclenchement d'une alarme si ces conditions sont inadéquates, voire simplement non optimales. En complément, la surveillance peut permettre une observation régulière de ces conditions de stockage.

Lesdites conditions sont définies par au moins une grandeur physique « ambiante », c'est-à-dire une grandeur physique de l'environnement du flacon 1. La ou les grandeurs ainsi surveillées sont avantageusement choisies parmi toutes combinaisons de la température, l'hygrométrie, la luminosité, et l'inclinaison du flacon 1. L'homme du métier saura utiliser si besoin d'autres grandeurs telles que la pression atmosphérique, ou l'altitude.

La présente invention se distingue en l'utilisation astucieuse d'équipements de surveillance 3 chacun dédié à un flacon 1.

En d'autres termes, si l'utilisateur stocke plusieurs flacons 1 avec des conditions optimales différentes (du fait de types différents de boissons alcoolisées par exemple), le présent procédé gère la surveillance indépendante de chacun des flacons via les équipements de surveillance 3.

Ainsi, peut tout à fait être détecté que les conditions ne conviennent plus à un premier flacon 1, tout en convenant pour un autre flacon 1.

Par ailleurs, il est à noter que plusieurs flacons 1 même stockés au même endroit (une cave par exemple), peuvent en fait subir des conditions de stockage différentes. Par exemple, il peut y avoir un gradient de température dans la pièce. En outre, différents flacons peuvent avoir des inclinaisons différentes.

Enfin, on comprendra que les conditions optimales peuvent pour un produit alimentaire donné tel qu'une boisson alcoolisée varier avec le temps.

L'utilisation d'équipements 3 individuels est ainsi particulièrement adaptée à la surveillance de bouteilles de grands vins ou champagnes, qui ont une valeur élevée, une longue période de garde, et une plus faible tolérance aux mauvaises conditions de stockage si l'on souhaite la dégustation parfaite.

Trois équipements sont plus précisément impliqués dans le présent procédé, en l'espèce :
- Le ou les équipements de surveillance 3, chacun comprenant au moins un capteur 30 d'une grandeur physique (i.e. comme expliqué un capteur de température, un capteur d'hygrométrie, un capteur de luminosité, un capteur d'inclinaison, ou un altimètre), avantageusement un capteur 30 de chaque type, et typiquement des moyens de traitement de données 31 (en particulier un processeur ou un microcontrôleur), des moyens de stockage de données 32 (notamment une mémoire, en particulier de type flash, et/ou la mémoire du microcontrôleur), des moyens de connexion sans fil 33 (en particulier de type WiFi comme l'on verra plus loin, mais également Bluetooth, avantageusement Bluetooth Low Energy (BLE), voire 3G/4G) pour connexion à un réseau 20, en particulier internet. L'équipement de surveillance 3 comprend également une batterie (soit à usage unique, soit rechargeable, et alors l'équipement 3 peut comprendre un port, par exemple micro-USB, à cet effet), et le cas échéant une interface minimale, par exemple un petit bouton poussoir d'initialisation (voir plus loin), et éventuellement un écran basique même si avantageusement il n'en comprend pas pour minimiser sa consommation. D'autres caractéristiques des équipements de surveillance 3 seront décrites plus loin.
- Un terminal mobile 2, typiquement de type smartphone ou tablette tactile. Il comprend ses propres moyens de traitement et de stockage de données, une interface telle qu'un écran tactile et des moyens de communication sans fil aptes à communiquer avec le réseau 20 et éventuellement directement avec les moyens de communication sans fil 33 de l'équipement de surveillance 3, par exemple via l'établissement d'un réseau WiFi ad-hoc.
- Un serveur 4 du réseau 20, c'est-à-dire un équipement fixe distant comprenant typiquement ses moyens de traitement de données et de stockage de données, en particulier comme on va le voir pour gérer une base de données.

### Procédé

Le présent procédé commence par une étape (a) d'association audit flacon 1 d'un équipement de surveillance 3, cette étape comprenant la transmission au serveur 4 de données descriptives dudit flacon 1 depuis le terminal mobile 2. Les données descriptives peuvent consister en une pluralité de données générales telles que l'année de production de la boisson alcoolisée, son type, son cépage, son volume, le producteur, etc., ou être directement une référence spécifique du flacon 1 permettant d'identifier son contenu, telle que le nom complet de la cuvée, mais également une photographie du code-barres voire de l'étiquette du flacon 1 (cas typique des bouteilles de vin/champagne). On connait en effet des mécanismes de reconnaissance d'image permettant au serveur 4 d'identifier le flacon 1 à partir d'une banque d'images. Comme l'on va voir, les données descriptives du flacon 1 vont permettre au serveur 4 de déterminer des gammes acceptables de valeurs des grandeurs physiques pour la conservation

A noter que cette étape peut être précédée d'un appariement du terminal mobile 2 avec ledit équipement de surveillance 3, à faire une fois à l'achat d'un équipement de surveillance 3. Cet appariement est une « initialisation » de l'équipement de surveillance 3, qui consiste par exemple à presser le petit bouton poussoir (qui peut être un capteur d'ouverture ou de fermeture du dispositif) pour activer un mode de configuration de l'équipement de surveillance 3, dans lequel une communication à courte portée est activée entre l'équipement de surveillance 3 et le terminal mobile 2 à proximité (par exemple via la génération d'un réseau WiFi avec un mot de passe prédéterminé par les moyens de communication 33 de l'équipement de surveillance 3, auquel le terminal va se connecter), de sorte à transmettre à l'équipement des informations telles qu'un identifiant unique de l'utilisateur/du terminal mobile 2 (par exemple un nom de compte, avantageusement un identifiant permettant de communiquer avec le terminal mobile 2, par exemple un numéro de téléphone, une adresse e-mail, etc.), et des informations de connexion (SSID et mot de passe d'un réseau WiFi auquel se connecter, etc.). De façon générale, on comprendra que l'homme du métier connait des procédures d'initialisation de périphériques sans fil et saura les utiliser pour le présent équipement de surveillance 3.

A noter que cette étape préalable d'appariement peut comprendre l'envoi au serveur 4 (par le terminal mobile 2 ou l'équipement de surveillance 3) d'un couple de leurs identifiants uniques de sorte que le terminal mobile 2 et l'équipement de surveillance 3 soient appariés aussi dans la base de données du serveur 4.

Une pluralité d'équipements de surveillance 3 peut être appariée avec un même terminal mobile 2.

Pour revenir à l'étape (a), l'association comprend typiquement la disposition de l'équipement de surveillance 3 à proximité du flacon 1 de sorte à ce que le ou les capteurs 30 de l'équipement de surveillance 3 puissent acquérir les grandeurs physiques de l'environnement.

Afin de mesurer les grandeurs physiques au plus près du flacon, l'association de l'équipement de surveillance 3 audit flacon 1 comprend même le montage physique dudit équipement de surveillance 3 sur le flacon 1, par exemple au sommet d'une bouteille de vin/champagne (de sorte à enserrer le col à la façon d'un muselet). En d'autres termes, l'équipement 3 se monte sur le goulot de la bouteille, et enserre le bouchon (i.e. le bouchon est « à l'intérieur » de l'équipement 3 lorsqu'il est monté). Cette position est très astucieuse car d'une part la fixation de l'équipement 3 est facile est fiable, ensuite l'équipement 3 ne gêne pas le stockage des flacons 1, et enfin le bouchon est le composant critique pour la bonne conservation du flacon et ainsi c'est le meilleur endroit où surveiller les grandeurs physiques, et en plus il est protégé.

L'équipement de surveillance 3 prend une forme sensiblement cylindrique comme celle d'un antivol de bouteille, avec des mâchoires assurant le verrouillage de l'équipement de surveillance 3 sur la bouteille. L'équipement de surveillance 3 est alors typiquement un boitier en plastique (ou en un autre matériau tel que du bois, de l'acier, etc.) enfermant de façon sécurisée les capteurs 30 et les modules électroniques (en protégeant ces derniers de l'atmosphère souvent humide des caves).

De façon préférée, lesdites mâchoires sont déformables et le montage sur le goulot ou le retrait se fait en forçant (ce provoque la déformation des mâchoires). Typiquement les mâchoires sont formées de bras équipés d'un ergot radial, et l'extrémité de ses bras s'écarte lors d'un tel ou montage ou retrait. De façon particulièrement l'équipement 3 présente en outre une enveloppe externe rigide elle-même cylindrique, apte à coulisser longitudinalement par-dessus le reste de l'équipement 3 et particulièrement les mâchoires. Ainsi, lorsque l'enveloppe recouvre les mâchoires, celles-ci ne peuvent pas s'écarter et l'équipement 3 est verrouillé (il ne peut pas être ni monté ni retiré), et lorsque l'enveloppe est tirée vers le haut et ne recouvre pas les bras, ceux-ci peuvent se déformer vers l'extérieur de sorte à permettre le montage ou le retrait. On comprendra que d'autres solutions de fixations sont possibles, par exemple l'appui sur un mouton qui verrouille /déverrouille tout déplacement ou déformation des mâchoires.

Lorsque l'équipement de surveillance 3 est monté sur le flacon 1, il devient solidaire de ce dernier. On note que la fixation peut être libérée en faisant une manipulation particulière, par exemple en faisant coulisser vers le haut l'enveloppe ou en appuyant sur un bouton, de sorte à accéder au bouchon si l'utilisateur veut déguster la boisson alcoolisée.

Le signalement auprès du serveur 4 de l'association audit flacon 1 d'un équipement de surveillance 3 peut être à l'initiative du terminal mobile 2 (l'utilisateur peut envoyer au serveur 4 un message depuis le terminal mobile 2 contenant un identifiant unique de l'équipement de surveillance 3 et les données descriptives du flacon 1), mais de façon préférée elle est à l'initiative de l'équipement de surveillance 3, en particulier si celui-ci se monte physiquement sur le flacon 1.

Dans ce dernier cas, l'équipement de surveillance 3 peut détecter son montage (par exemple via le déplacement ou la déformation des mâchoires susmentionnées, ou via un contacteur placé entre les mâchoires), et envoyer en conséquence un message d'initialisation de la surveillance du flacon 1 au serveur 4, ce message comprenant avantageusement son identifiant unique.

Ainsi, le serveur 4 recevant le message peut déterminer grâce à sa base de données le terminal mobile 2 apparié, et lui envoyer une requête desdites informations de données descriptives dudit flacon 1, à laquelle l'utilisateur va répondre en envoyant les données requises.

A réception des données descriptives dudit flacon 1, le serveur 4 complète sa base de données, de sorte à associer l'équipement de surveillance 3 ces données descriptives et avantageusement un horodatage de début de la surveillance (correspondant avantageusement au moment de réception du message d'initialisation de la surveillance). Comme expliqué précédemment, les données descriptives sont avantageusement traitées de sorte à identifier le flacon 1 et son contenu si nécessaire (par exemple si les données descriptives sont un code-barres). Des traitements supplémentaires peuvent être mis en œuvre et seront évoqués plus loin.

### Surveillance

Le procédé comprend une étape (b) de réception périodique par le serveur 4 de données acquises par l'au moins un capteur 30, en d'autres termes de données représentatives des valeurs des grandeurs physiques mesurées par les capteurs 30. Plus précisément, après son association au flacon 1 l'équipement de surveillance 3 s'active pour faire remonter les valeurs des grandeurs physiques ambiantes, soit directement via la connexion au réseau 20 (notamment via le point d'accès Wifi renseigné à l'initialisation), soit via le terminal mobile 2.

Les valeurs remontées sont stockées dans la base de données du serveur 4 pour analyse, et peuvent être accessibles à tout moment sur requête du terminal mobile 2.

A noter que la surveillance peut durer des années, donc la fréquence est avantageusement faible (par exemple une fois par jour) pour minimiser la consommation et prolonger la durée de vie de la batterie. En effet, il est souhaitable que la batterie soit suffisante pour faire fonctionner l'équipement de surveillance 3 sur toute la durée de stockage du flacon 1, qui peut durer des années. Une recharge intermédiaire ne serait pas pratique pour l'utilisateur, et nécessiterait de recommencer la procédure d'association.

Plus précisément, entre deux acquisitions l'équipement de surveillance 3 est préférentiellement quasiment intégralement éteint (à une horloge près), les capteurs 30 et/ou les moyens de communication 33 étant allumés ponctuellement. Dans la mesure où c'est l'émission des données qui consomme le plus d'énergie, il est possible de mettre en œuvre un mode hybride dans lequel les acquisitions de données sont faites à une première fréquence (par exemple toutes les six heures), et les transmissions à une deuxième fréquence inférieure (par exemple tous les jours). Entre temps, les valeurs des grandeurs sont stockées sur la mémoire 2, et envoyées en batch.

A noter que si l'utilisateur requiert sur son terminal mobile 2 les valeurs des grandeurs à un instant donnée, le serveur 4 peut émettre une requête à l'équipement de surveillance 3 pour avoir une acquisition ponctuelle (supplémentaire par rapport à la périodicité) des valeurs au moment de la requête.

De façon particulièrement préférée, l'étape (a) comprend la détermination subséquente par le serveur 4 à partir des données descriptives dudit flacon 1 d'une fréquence de mise en œuvre de l'étape (b), ladite fréquence de mise en œuvre étant fonction d'une durée théorique de vieillissement du produit alimentaire du flacon 1. Plus précisément, l'étape (a) peut comprendre la détermination de durée théorique de vieillissement du produit alimentaire du flacon 1 en fonction desdites données représentatives remontées (par exemple par consultation d'une banque de flacons 1 chacun associé à une durée de vieillissement théorique, et comme on le verra, des valeurs théoriques optimales des grandeurs physiques surveillées).

Et plus la durée de vieillissement déterminée est longue, plus la fréquence peut être faible. L'idée est de contrôler le nombre total d'acquisitions de sorte à optimiser la consommation énergétique sur toute la durée de vieillissement.

En effet, si la durée de vieillissement est longue, il faut économiser davantage l'énergie en limitant le nombre de transmission, d'où une réduction proportionnelle de la fréquence. La détermination de cette fréquence de mise en œuvre en fonction d'une durée théorique de vieillissement du produit alimentaire du flacon 1 permet de garantir que l'équipement de surveillance 3 ne tombera pas à cours d'énergie (batterie vide) sur la vie du flacon 1.

A noter qu'en cas de dégradation des conditions de stockage (voir plus loin), la fréquence peut être augmentée temporairement (on remplace la fréquence déterminée pour le flacon par une fréquence prédéterminée dite de sécurité, par exemple toutes les heures), de sorte à surveiller le rétablissement des conditions nominales. Lorsque c'est le cas, on rebascule sur la fréquence déterminée. A noter que ce mode de sécurité peut entraîner la nécessité d'un rechargement de la batterie durant la durée de vieillissement théorique du fait de l'augmentation imprévue de la sollicitation des capteurs 30 de l'équipement de surveillance 3.

### Notification

Dans une étape (c), mise en œuvre par le serveur 4, est générée et émise une notification au terminal mobile 2, en fonction desdites données acquises par l'au moins un capteur 30 et des données descriptives dudit flacon 1.

De façon typique, le procédé comprend plus précisément, suite à l'étape (a) la détermination de valeurs de référence des grandeurs physiques mesurées par les capteurs 30 en fonction des données descriptives dudit flacon 1, en d'autres termes des valeurs « nominales » des grandeurs physiques pour la conservation optimales du flacon 1. Pour cela, comme pour la durée de vieillissement, le serveur 4 peut par exemple consulter une banque de flacons 1 chacun associé à un ensemble de valeurs de référence des grandeurs physiques, et/ou des gammes de valeurs acceptables. Comme expliqué, ces valeurs de référence peuvent évoluer en fonction du vieillissement, et donc il peut être prévu de les re-déterminer régulièrement.

Les notifications générées peuvent quant à elles prendre plusieurs formes.

Dans une premier mode de réalisation, la notification est un compte-rendu périodique, et l'étape (c) est mise en œuvre à une certaine périodicité (qui peut être égale ou différente des périodicités d'acquisition et/ou d'envoi des données depuis l'équipement), par exemple une fois par semaine. Cette notification peut être globale pour tous les équipements de surveillance 3 appariés au terminal mobile 2.

Cette notification peut comprendre les valeurs moyennes et/ou les valeurs extrémales constatées des grandeurs physiques sur la période, ainsi que les valeurs de référence correspondantes, de sorte à identifier quels flacons ont une meilleure conservation que d'autres, et si des actions sont nécessaires.

La notification peut également rappeler la durée de vieillissement théorique et calculer combien de cette durée il reste (depuis l'association de l'équipement de surveillance 3 avec le flacon 1 - et plus précisément la réception du message d'initialisation de la surveillance - dont on fait l'hypothèse qu'elle est au moment de la mise en cave).

Dans un second mode de réalisation (qui peut être alternatif ou complémentaire avec le premier mode), l'étape (c) est mise en œuvre si un écart entre lesdites valeurs mesurées par l'au moins un capteur 30 et les valeurs de référence est supérieur à un seuil prédéterminé. Ce seuil peut être une valeur fixe (par exemple 5°C), ou un ratio.

Il s'agit d'un mode alarme, qui prévient l'utilisateur que les conditions de stockage se dégradent, et qu'il faut agir sous peine de perdre les qualités gustatives de la boisson alcoolisée contenue dans le flacon 1.

A noter que ce mode alarme peut aussi être utilisé à l'expiration de la durée de vieillissement théorique (de sorte à suggérer à l'utilisateur de déguster rapidement la boisson alcoolisée qui est alors à son apogée). Il peut être prévu que l'utilisateur reçoive en même temps des conseils de dégustation, des recommandations, des accords mets-et-vins, etc.

Par ailleurs, comme expliqué précédemment, une notification peut également être envoyée à l'équipement de surveillance 3 à l'origine de l'alarme de sorte à activer un mode de sécurité dans lequel une fréquence de mise à jour déterminée pour le flacon 1 est remplacée par une fréquence de sécurité jusqu'à fin de l'épisode de conditions dégradées (écart entre lesdites données acquises par l'au moins un capteur 30 et les valeurs de référence fonction des données descriptives dudit flacon 1 supérieur à un seuil prédéterminé) : lorsque l'utilisateur a agi et que les valeurs des grandeurs physiques mesurées sont à nouveau nominales, le serveur 4 envoie une nouvelle notification de fin du mode de sécurité et de retour à la fréquence déterminée pour économiser la batterie. Ladite notification est une requête de modification temporaire de la fréquence de mise à jour.

On comprendra que l'alarme peut être déclenchée (et une notification émise) si l'état de la batterie se dégrade plus vite que prévu (par exemple si le mode de sécurité avait dû être activé, ou si la batterie est défectueuse). L'utilisateur averti sur son terminal mobile 2 doit donc aller recharger la batterie (par exemple en connectant sur place son terminal mobile 2 à l'équipement de surveillance 3 via l'éventuel port, car effet il n'y a pas toujours d'électricité dans les espaces de stockage de boissons alcoolisées tels qu'une cave), ou la changer, de sorte à éviter de perdre l'historique de surveillance et de devoir faire une nouvelle initialisation.

### Equipement de surveillance

L'équipement de surveillance 3 pour la mise en œuvre du procédé selon le premier aspect est comme expliqué adapté pour être associé à, et plus précisément monté sur un flacon 1 (via des moyens de fixation dédiés tels que des mâchoires), il comprend des moyens de traitement de données 31, avantageusement des moyens de stockage de données 32, des moyens de connexion sans fil 33 à un réseau 20 (et via là à un terminal mobile 2 et un serveur 4, et au moins un capteur 30 d'une grandeur physique de l'environnement.

Les moyens de traitement de données 31 mettent en œuvre :
- Un module d'association de l'équipement de surveillance 3 au flacon 1 de produit alimentaire (typiquement sur détection d'un montage de l'équipement de surveillance 3 sur le flacon 1) ;
- Un module d'émission périodique vers un serveur 4 de données acquises par l'au moins un capteur 30 (à une fréquence régulière).

Selon un deuxième aspect est proposé le système comprenant en outre le serveur 4, auquel l'équipement de surveillance 3 est connecté via le réseau 20. Le système peut comprendre une pluralité d'équipements 3.

Le serveur 4 est en outre configuré pour mettre en œuvre un module de génération et d'émission d'une notification vers un terminal mobile 2 (celui ayant émis des données descriptives dudit flacon 1), en fonction desdites données acquises par l'au moins un capteur 30 et des données descriptives dudit flacon 1 reçues depuis le terminal mobile 2.

Avantageusement, le système comprenant en outre le terminal mobile 2.

Le terminal mobile 2 et l'équipement de surveillance 3 sont préférentiellement configurés pour mettre en œuvre un module d'appariement du terminal mobile 2 avec ledit équipement de surveillance 3.

A noter que le système peut comprendre une pluralité de terminaux mobile 2 chacun apparié à un ou plusieurs équipements de surveillance 3.

L'équipement de surveillance 3 est comme expliqué adapté pour être associé à, et plus précisément monté sur un flacon 1 (par exemple via des moyens de fixation dédiés tels que des mâchoires), il comprend des moyens de traitement de données 31, avantageusement des moyens de stockage de données 32, des moyens de connexion sans fil 33 à un réseau 20 (et via là à un terminal mobile 2 et un serveur 4, et au moins un capteur 30 d'une grandeur physique de l'environnement.

Les moyens de traitement de données 31 mettent en œuvre :
- Un module d'association de l'équipement de surveillance 3 au flacon 1 de produit alimentaire (typiquement sur détection d'un montage de l'équipement de surveillance 3 sur le flacon 1) ;
- Un module d'émission périodique vers un serveur 4 de données acquises par l'au moins un capteur 30 (à une fréquence régulière).

Selon un troisième aspect est proposé le système comprenant en outre le serveur 4, auquel l'équipement de surveillance 3 est connecté via le réseau 20. Le système peut comprendre une pluralité d'équipements 3.

Le serveur 4 est en outre configuré pour mettre en œuvre un module de génération et d'émission d'une notification vers un terminal mobile 2 (celui ayant émis des données descriptives dudit flacon 1), en fonction desdites données acquises par l'au moins un capteur 30 et des données descriptives dudit flacon 1 reçues depuis le terminal mobile 2.

Avantageusement, le système comprenant en outre le terminal mobile 2.

Le terminal mobile 2 et l'équipement de surveillance 3 sont préférentiellement configurés pour mettre en œuvre un module d'appariement du terminal mobile 2 avec ledit équipement de surveillance 3.

A noter que le système peut comprendre une pluralité de terminaux mobile 2 chacun apparié à un ou plusieurs équipements de surveillance 3.

## Revendications

1. Procédé de surveillance d'un flacon (1) de produit alimentaire, le produit alimentaire étant une boisson alcoolisée et le flacon (1) étant une bouteille bouchée par un bouchon enfoncée dans un goulot, le procédé comprenant l'étape de :
(a) Association audit flacon (1) d'un équipement de surveillance (3) comprenant au moins un capteur (30) d'une grandeur physique, et étant **caractérisé en ce qu'**il comprend les étapes de :
(a') transmission à un serveur (4) de données descriptives dudit flacon (1) depuis un terminal mobile (2) ;
(b) Réception périodique par le serveur (4) de données acquises par l'au moins un capteur (30) ;
(c) En fonction desdites données acquises par l'au moins un capteur (30) et des données descriptives dudit flacon (1), génération et émission d'une notification au terminal mobile (2) ;
dans lequel l'association de l'équipement de surveillance (3) audit flacon (1) comprend le montage physique dudit équipement de surveillance (3) sur le flacon (1), l'équipement de surveillance (3) présentant une forme sensiblement cylindrique et étant monté sur le goulot de la bouteille en enserrant le bouchon, l'équipement de surveillance (3) présentant des mâchoires assurant le verrouillage de l'équipement de surveillance (3) sur la bouteille.

2. Procédé selon la revendication 1, comprenant une étape préalable d'appariement du terminal mobile (2) avec ledit équipement de surveillance (3).

3. Procédé selon l'une des revendications 1 et 2, dans lequel l'association de l'équipement de surveillance (3) audit flacon (1) comprend la détection par ledit équipement de surveillance (3) de son montage physique sur le flacon (1), et l'envoi d'un message d'initialisation de la surveillance du flacon (1) au serveur (4).

4. Procédé selon l'une des revendications 1 à 3, dans lequel le ou les capteurs (30) sont choisis parmi un capteur de température, un capteur d'hygrométrie, un capteur de luminosité et un capteur d'inclinaison.

5. Procédé selon l'une des revendications 1 à 4, dans lequel l'étape (a) comprend la détermination subséquente par le serveur (4) à partir des données descriptives dudit flacon (1) d'une fréquence de mise en oeuvre de l'étape (b), ladite fréquence de mise en oeuvre étant fonction d'une durée théorique de vieillissement du produit alimentaire du flacon (1).

6. Procédé selon la revendication 5, dans lequel l'étape (c) comprend en outre la génération et l'émission d'une notification à l'équipement de surveillance (3) de sorte à modifier temporairement ladite fréquence de mise en œuvre de l'étape (b).

7. Procédé selon l'une des revendications 1 à 6, dans lequel l'étape (c) est mise en œuvre si un écart entre lesdites données acquises par l'au moins un capteur (30) et des valeurs de référence fonction des données descriptives dudit flacon (1) est supérieur à un seuil prédéterminé.

8. Système comprenant un équipement de surveillance (3), l'équipement de surveillance (3) comprenant au moins un capteur (30) d'une grandeur physique et des moyens de traitement de données (31), le système étant **caractérisé en ce qu'**il comprend un serveur (4) connecté à l'équipement de surveillance (3) via un réseau (20), les moyens de traitement de données (31) mettant en oeuvre :
- Un module d'association de l'équipement de surveillance (3) à un flacon (1) de produit alimentaire, le produit alimentaire étant une boisson alcoolisée et le flacon (1) étant une bouteille bouchée par un bouchon enfoncée dans un goulot, l'association de l'équipement de surveillance (3) audit flacon (1) comprenant le montage physique dudit équipement de surveillance (3) sur le flacon (1), l'équipement de surveillance (3) présentant une forme sensiblement cylindrique avec des mâchoires assurant le verrouillage de l'équipement de surveillance (3) sur la bouteille, et étant monté sur le goulot de la bouteille en enserrant le bouchon, des mâchoires assurant le verrouillage de l'équipement de surveillance (3) sur la bouteille ;
- Un module d'émission périodique vers le serveur (4) de données acquises par l'au moins un capteur (30) ;
le serveur (4) étant en outre configuré pour mettre en œuvre un module de génération et d'émission d'une notification vers un terminal mobile (2), en fonction desdites données acquises par l'au moins un capteur (30) et de données descriptives dudit flacon (1) reçues depuis le terminal mobile (2).

9. Système selon la revendication 8, comprenant en outre le terminal mobile (2), le terminal mobile (2) et l'équipement de surveillance (3) étant configurés pour mettre en œuvre un module d'appariement du terminal mobile (2) avec ledit équipement de surveillance (3).

## Patentansprüche

1. Verfahren zur Überwachung eines Behälters (1) eines Lebensmittelprodukts, wobei das Lebensmittelprodukt ein alkoholhaltiges Getränk ist und der Behälter (1) eine Flasche ist, die mit einem Stopfen verschlossen ist, der in einen Flaschenhals geschoben wird, wobei das Verfahren den Schritt umfasst zum:
(a) Zuordnen zum Behälter (1) einer Überwachungsausrüstung (3), die mindestens einen Sensor (30) einer physikalischen Größe umfasst, und **dadurch gekennzeichnet, dass** es die Schritte umfasst zum:
(a') Übertragen an einen Server (4) von Beschreibungsdaten des Behälters (1) von einem mobilen Endgerät (2);
(b) Periodisches Empfangen durch den Server (4) von durch den mindestens einen Sensor (30) erfassten Daten;
(c) In Abhängigkeit von den durch den mindestens einen Sensor (30) erfassten Daten und den Beschreibungsdaten des Behälters (1), Erzeugen und Senden einer Benachrichtigung an das mobile Endgerät (2);
wobei die Zuordnung der Überwachungsausrüstung (3) zum Behälter (1) die physische Montage der Überwachungsausrüstung (3) an dem Behälter (1) umfasst, die Überwachungsausrüstung (3) eine im Wesentlichen zylindrische Form aufweist, und an dem Flaschenhals der Flasche montiert ist, indem sie den Stopfen einklemmt, wobei die Überwachungsausrüstung (3) Klemmbacken aufweist, die für die Verriegelung der Überwachungsausrüstung (3) an der Flasche sorgen.

2. Verfahren nach Anspruch 1, einen vorherigen Schritt des Paarens des mobilen Endgeräts (2) mit der Überwachungsausrüstung (3) umfassend.

3. Verfahren nach einem der Ansprüche 1 und 2, wobei die Zuordnung der Überwachungsausrüstung (3) zum Behälter (1) das Erkennen durch die Überwachungsausrüstung (3) ihrer physischen Montage an dem Behälter (1), und das Schicken einer Initialisierungsnachricht der Überwachung des Behälters (1) an den Server (4) umfasst.

4. Verfahren nach einem der Ansprüche 1 bis 3, wobei der oder die Sensoren (30) aus einem Temperatursensor, einem Feuchtigkeitssensor, einem Helligkeitssensor und einem Neigungssensor ausgewählt sind.

5. Verfahren nach einem der Ansprüche 1 bis 4, wobei der Schritt (a) die nachfolgende Bestimmung durch den Server (4) aus den Beschreibungsdaten des Behälters (1) einer Häufigkeit der Umsetzung des Schrittes (b) umfasst, wobei die Häufigkeit der Umsetzung von einer theoretischen Alterungsdauer des Lebensmittelproduktes des Behälters (1) abhängig ist.

6. Verfahren nach Anspruch 5, wobei der Schritt (c) weiter das Erzeugen und das Senden einer Benachrichtigung an die Überwachungsausrüstung (3) umfasst, um vorübergehend die Häufigkeit der Umsetzung des Schrittes (b) zu ändern.

7. Verfahren nach einem der Ansprüche 1 bis 6, wobei der Schritt (c) umgesetzt wird, wenn eine Abweichung zwischen den durch den mindestens einen Sensor (30) erfassten Daten und von Referenzwerten, die von den Beschreibungsdaten des Behälters (1) abhängig sind, größer als ein vorbestimmter Schwellenwert ist.

8. System, eine Überwachungsausrüstung (3) umfassend, wobei die Überwachungsausrüstung (3) mindestens einen Sensor (30) für eine physikalische Größe und Datenverarbeitungsmittel (31) umfasst, wobei das System **dadurch gekennzeichnet ist, dass** es einen Server (4) umfasst, der über ein Netzwerk (20) mit der Überwachungsausrüstung (3) verbunden ist, wobei die Datenverarbeitungsmittel (31) Folgendes anwenden:
- ein Zuordnungsmodul der Überwachungsausrüstung (3) zu einem Behälter (1) eines Lebensmittelprodukts, wobei das Lebensmittelprodukt ein alkoholhaltiges Getränk ist und der Behälter (1) eine Flasche ist, die mit einem Stopfen verschlossen ist, der in den Flaschenhals geschoben wird, wobei die Zuordnung der Überwachungsausrüstung (3) zum Behälter (1) die physische Montage der Überwachungsausrüstung (3) an dem Behälter (1) umfasst, wobei die Überwachungsausrüstung (3) eine im Wesentlichen zylindrische Form mit Klemmbacken aufweist, die für die Verriegelung der Überwachungsausrüstung (3) an der Flasche sorgen, und an dem Flaschenhals der Flasche montiert sind, indem sie den Stopfen einklemmen, wobei die Klemmbacken für die Verriegelung der Überwachungsausrüstung (3) an der Flasche sorgen;
- ein Modul zum periodischen Senden zum Server (4) von durch den mindestens einen Sensor (30) erfassten Daten;
wobei der Server weiter konfiguriert ist, um ein Modul zum Erzeugen und Senden einer Benachrichtigung zu einem mobilen Endgerät (2) in Abhängigkeit von den von dem mindestens einen Sensor (30) erfassten Daten und den Beschreibungsdaten des Behälters (1) anwendet, die von dem mobilen Endgerät (2) empfangen werden.

9. System nach Anspruch 8, weiter das mobile Endgerät (2) umfassend, wobei das mobile Endgerät (2) und die Überwachungsausrüstung (3) konfiguriert sind, um ein Paarungsmodul des mobilen Endgeräts (2) mit der Überwachungsausrüstung (3) anzuwenden.

## Claims

1. A method for monitoring a food product vessel (1), the food product being an alcoholic beverage and the vessel (1) being a bottle plugged by a plug pressed into a bottleneck, the method comprising the steps of:
(a) Associating a monitoring equipment (3) comprising at least one sensor (30) of a physical quantity to said vessel (1), and being **characterised in that** it comprises the steps of:
(a') Transmitting descriptive data of said vessel (1) to a server (4) from a mobile terminal (2);
(b) The server (4) periodically receiving data acquired by the at least one sensor (30);
(c) According to said data acquired by the at least one sensor (30) and the descriptive data of said vessel (1), generating and emitting a notification to the mobile terminal (2);
wherein the association of the monitoring equipment (3) to said vessel (1) comprises the physical mounting of said monitoring equipment (3) on the vessel (1), the monitoring equipment (3) having a substantially cylindrical shape and being mounted on the bottleneck of the bottle by clasping the plug, the monitoring equipment (3) having jaws ensuring the locking of the monitoring equipment (3) on the bottle.

2. The method according to claim 1, comprising a prior step of pairing the mobile terminal (2) with said monitoring equipment (3).

3. The method according to one of claims 1 and 2, wherein the association of the monitoring equipment (3) to said vessel (1) comprises said monitoring equipment (3) detecting the physical mounting thereof on the vessel (1), and sending a message for initialising the monitoring of the vessel (1) to the server (4).

4. The method according to one of claims 1 to 3, wherein the sensor(s) (30) are selected among a temperature sensor, a hygrometry sensor, a luminosity sensor and an inclination sensor.

5. The method according to one of claims 1 to 4, wherein step (a) comprises the server (4) subsequently determining from the descriptive data of said vessel (1) a frequency of implementation of step (b), said implementation frequency depending on a theoretical ageing duration of the food product of the vessel (1).

6. The method according to claim 5, wherein step (c) further comprises generating and emitting a notification to the monitoring equipment (3) so as to temporarily modify said frequency of implementation of step (b).

7. The method according to one of claims 1 to 6, wherein step (c) is implemented if a discrepancy between said data acquired by the at least one sensor (30) and reference values depending on the descriptive data of said vessel (1) is greater than a predetermined threshold.

8. A system comprising a monitoring equipment (3), the monitoring equipment (3) comprising at least one sensor (30) of a physical quantity and data processing means (31), the system being **characterised in that** it comprises a server (4) connected to the monitoring equipment (3) via a network (20), the data processing means (31) implementing:
- A module for associating the monitoring equipment (3) to a food product vessel (1), the food product being an alcoholic beverage and the vessel (1) being a bottle plugged by a plug pressed into a bottleneck, the association of the monitoring equipment (3) to said vessel (1) comprising the physical mounting of said monitoring equipment (3) on the vessel (1), the monitoring equipment (3) having a substantially cylindrical shape with jaws ensuring the locking of the monitoring equipment (3) on the bottle, and being mounted on the bottleneck of the bottle by clasping the plug, jaws ensuring the locking of the monitoring equipment (3) on the bottle;
- A module for periodically emitting towards the server (4) data acquired by the at least one sensor (30);
the server (4) being further configured to implement a module for generating and emitting a notification towards a mobile terminal (2), according to said data acquired by the at least one sensor (30) and descriptive data of said vessel (1) received from the mobile terminal (2).

9. The system according to claim 8, further comprising the mobile terminal (2), the mobile terminal (2) and the monitoring equipment (3) being configured to implement a module for pairing the mobile terminal (2) with said monitoring equipment (3).
